# EUROPEAN PATENT SPECIFICATION

(11) **EP 4 495 574 B1**
(45) Date of publication and mention of the grant of the patent: **02.09.2026**
(21) Application number: 22932226.8
(22) Date of filing: 18.03.2022
(51) Int. Cl.: G01N 5/02, G01N 29/02, G01N 29/036, G01N 33/00

(54) **SUBSTANCE DETECTING SYSTEM**
SUBSTANZNACHWEISSYSTEM
SYSTÈME DE DÉTECTION DE SUBSTANCES

(43) Date of publication of application: 22.01.2025
(73) Proprietor: I-PEX Inc., Fushimi-ku, Kyoto-shi Kyoto 612-8024 (JP)
(72) Inventor: OGATA Kenji, Ogori-shi, Fukuoka 838-0106 (JP); KUROGI Shogo, Ogori-shi, Fukuoka 838-0106 (JP)
(74) Representative: SSM Sandmair
(86) International application number: PCT/JP2022/012794
(87) International publication number: WO 2023/175956

(56) References cited:
- WO-A1-2021/053410
- WO-A1-2021/172592
- WO-A1-2021/172592
- JP-A- 2009 216 672
- JP-A- 2010 263 365
- JP-A- 2018 025 418
- JP-A- 2021 165 730
- JP-A- 2022 010 352
- JP-A- H03 282 232
- US-A1- 2010 277 251

## Description

### Technical Field

The present disclosure relates to a substance detecting system.

### Background Art

In Patent Literature 1, a chemical sensor device serving as a substance detecting system that detects a substance, based on the amount of change in resonance frequency that occurs when the substance adsorbs to or desorbs from a sensitive film is disclosed. The chemical sensor device includes a plurality of oscillators on each of which a sensitive film exhibiting adsorption/desorption characteristics for a different substance is installed. Each of the oscillator includes a piezoelectric substrate and is vibrated by the piezoelectric substrate to which AC voltage is applied deforming. When a substance adsorbs to or desorbs from the sensitive film, resonance frequency of each oscillator changes. This configuration enables detection of a substance.

Use of the chemical sensor device enables an odor comprising a plurality of types of substances to be detected. An odor contained in gas is identified based on a pattern of reaction values of the respective sensitive films, that is, a composition ratio of a plurality of substances causing the odor.

### Citation List

### Patent Literature

Patent Literature 1: Unexamined Japanese Patent Application Publication No. 2009-204584

### Summary of Invention

### Technical Problem

As described above, an odor of gas comprises a plurality of types of substances contained in the gas. Therefore, in order to accurately identify an odor of gas, a plurality of substance sensors each of which detects one of the corresponding substances is required. In this case, a region in which the plurality of substance sensors is arranged is required, and there has been an inconvenience that the larger the number of types of substances to be detected becomes, the larger the area of the region becomes.

WO 2021/053410 A1 relates to a device for determining the physical parameters and chemical composition of a gas by means of measuring the flow of that gas.

The present disclosure has been made in consideration of the above-described circumstances, and an objective of the present disclosure is to provide a substance detecting system capable of reducing an area of a region required for arranging a plurality of substance sensors.

### Solution to Problem

In order to achieve the above-described objective, a substance detecting system according to the present disclosure is defined by independent apparatus claim 1.

The substance detecting system is configured such that
the chamber is constructed by substrates each having a communication hole being stacked in such a way that the communication holes communicate with each other,
a plurality of the substrates among the stacked substrates is first substrates each of which has a beam that closes a portion of a first communication hole serving as the communication hole, and
each of the substance sensors detects the substance, based on a change in a vibration frequency of the beam due to reaction with the substance.

The substance detecting system may be configured such that
a plurality of the substrates that is the stacked substrates except the first substrates is second substrates each of which has a second communication hole serving as the communication hole, and
the second substrates and the first substrates are alternately stacked in this order in the flow direction.

The substance detecting system may be configured such that
a substance reaction film that reacts with the substance is disposed on the beam, and
each of the second communication holes is shaped in a nozzle in such a way as to cause the gas to flow out toward the substance reaction film.

The substance detecting system is configured such that directions of the beams when viewed in the flow direction are different from each other between the beams adjacent to each other in the flow direction.

The substance detecting system may be configured such that
a shape of the beam when viewed in the flow direction is rotationally symmetric about a central axis of the cylindrical first communication hole, and
regarding the beams adjacent to each other in the flow direction, a direction of one of the beams when viewed in the flow direction points in a direction rotated about the central axis with respect to a direction of the other of the beams.

The substance detecting system may be configured such that
each of the beams has a cross shape when viewed in the flow direction, and
an angle at which, of the beams adjacent to each other in the flow direction, a central line of one of the beams and a central line of the other of the beams cross each other at the central axis is 45 degrees.

The substance detecting system may be configured such that a plurality of the chambers is arranged in parallel with one another.

The substance detecting system may be configured to include
a rectifier to control flows of the gas flowing into a plurality of the chambers into uniform flows and feeds the flows.

The substance detecting system may be configured such that the rectifier rectifies a flow of the gas in such a way that at least one of a flow rate and flow velocity of the gas that flows into respective ones of a plurality of the chambers is made uniform.

The substance detecting system may be configured such that a plurality of the chambers is arranged in such a manner as to have a same shape and size.

The substance detecting system may be configured such that
the feeder
is a pump, and
is installed on at least one of an upstream side of the inlet and a downstream side of the outlet.

The substance detecting system may be configured such that
the feeder
is a pump, and
is installed on both an upstream side of the inlet and a downstream side of the outlet.

The substance detecting system may be configured such that in a plurality of the substance sensors arranged in the chamber, substance sensors detecting a same substance are included.

The substance detecting system may be configured such that in a plurality of the substance sensors arranged in the chamber, substance sensors each detecting a different substance are included.

The substance detecting system may be configured such that the substance sensors correct detected values, based on correction values that increase in the flow direction.

According to the present disclosure, a plurality of the substance sensors is arranged in the flow direction of the gas from the inlet toward the outlet of the chamber. Thus, an area of a region required for arranging a plurality of substance sensors can be reduced.

### Brief Description of Drawings

FIG. 1 is a schematic diagram illustrating a basic configuration of a substance detecting system according to Embodiment 1 of the present disclosure;
FIG. 2 is a cross-sectional view illustrating a detailed configuration of the substance detecting system in FIG. 1;
FIG. 3 is a perspective view of a first substrate constituting the substance detecting system in FIG. 1;
FIG. 4 is a perspective view of a second substrate constituting the substance detecting system in FIG. 1;
FIG. 5 is a perspective view of a rectifier;
FIG. 6A is a diagram of a beam when viewed from an outlet side;
FIG. 6B is a diagram of the beam when viewed from an inlet side;
FIG. 7 is a cross-sectional view illustrating a first example of an overall configuration of the substance detecting system in FIG. 1;
FIG. 8 is a cross-sectional view illustrating a second example of the overall configuration of the substance detecting system in FIG. 1;
FIG. 9 is a timing chart illustrating operation of the substance detecting system in FIG. 1;
FIG. 10 is a top view illustrating an arrangement of beams constituting a substance detecting system according to Embodiment 2 of the present disclosure;
FIG. 11A is a diagram, which does not belong to the claimed invention, illustrating a flow of gas when directions of beams are aligned with each other;
FIG. 11B is a diagram, which belongs to the claimed invention, illustrating a flow of the gas when the directions of the beams are different from each other;
FIG. 12 is a schematic diagram illustrating a basic configuration of a substance detecting system according to Embodiment 3 of the present disclosure; and
FIG. 13 is a schematic diagram illustrating an arrangement of substance sensors detecting different types of substances.

### Description of Embodiments

Embodiments of the present disclosure are described below in detail with reference to the drawings. In the drawings, the same or equivalent parts are designated by the same reference numerals.

Embodiment 1, which does not belong to the claimed invention.

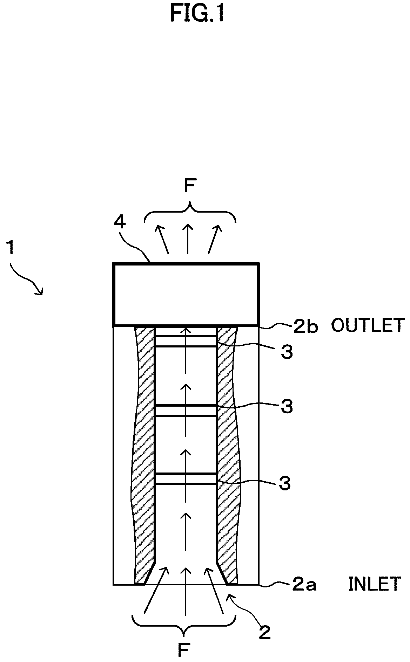

First, Embodiment 1 of the present disclosure is described. As illustrated in FIG. 1, a substance detecting system 1 according to the present embodiment detects a substance contained in ambient gas F. A substance to be detected is a substance constituting an odor of the gas F. The substance detecting system 1 detects a plurality of type of substances constituting an odor of the gas F. The substance detecting system 1 includes a chamber 2, substance sensors 3, and a feeder 4. The foregoing components are basic constituent components of the substance detecting system 1.

The chamber 2 causes the gas F to flow in from an inlet 2a and also discharges the gas F from an outlet 2b. The chamber 2 is a containing chamber of the gas F that restricts an inflow of the gas F in such a way that a state of the gas F to be contained inside the chamber 2 is brought into a steady state. As used herein, the steady state means a state in which flow of the gas F is always constant. More specifically, the steady state means a state in which direction of the flow, flow velocity, and a flow rate of the gas F fall within certain ranges and the gas F can be considered to be kept in the same state. The gas F in the chamber 2 is only required to be in the steady state when a substance is detected.

The substance sensors 3 detect substances that are contained in the gas F contained in the chamber 2. The substance detecting system 1 includes a plurality of substance sensors 3, and each of the substance sensors 3 detects a different substance. The plurality of substance sensors 3 is arranged in a flow direction of the gas F (hereinafter, simply referred to as "flow direction") from the inlet 2a toward the outlet 2b of the chamber 2. The substance sensors 3 are installed in the chamber 2 while the substance sensors 3 themselves do not completely close a flow path of the gas F from the inlet 2a to the outlet 2b.

The feeder 4 feeds the gas F into the chamber 2. The feeder 4 is, for example, a pump. When the feeder 4 operates, the gas F flows into the chamber 2 and is contained in the chamber 2. As a result, the plurality of substance sensors 3 detects a plurality of substances contained in the gas F and outputs signals corresponding to detection results of substances that the respective substance sensors 3 detected. Substances contained in the gas F are detected based on the output signals, and an odor of the gas F is identified based on a combination of the detected substances.

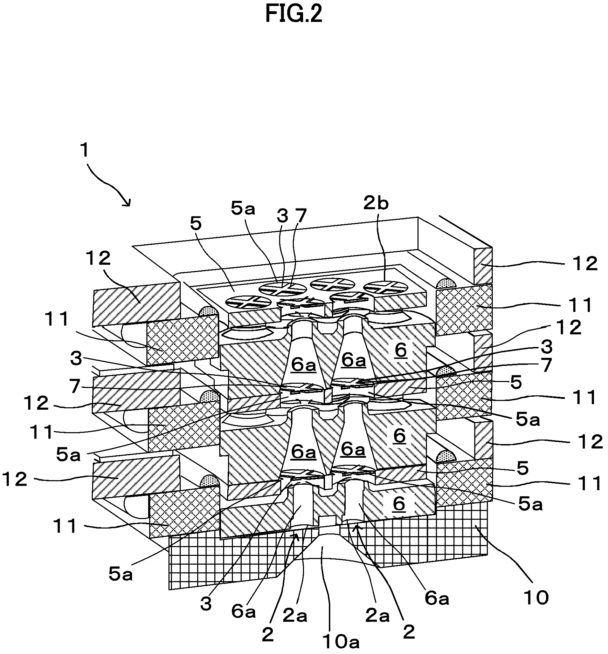

A detailed configuration of the substance detecting system 1 is described. As illustrated in FIG. 2, in practice, the substance detecting system 1 has a configuration in which a plurality of substrates is stacked. Specifically, the substance detecting system 1 has a configuration in which second substrates 6 and first substrates 5 are alternately stacked in this order in the flow direction from the inlet 2a toward the outlet 2b. In the present embodiment, the number of stacked second substrates 6 is three, and the number of stacked first substrates 5 is three. The first substrates 5 and the second substrates 6 are held by substrates 11 and 12 that are likewise stacked. Note that stacked substrates are referred to as a first tier, a second tier, and a third tier in the direction from the inlet 2a toward the outlet 2b.

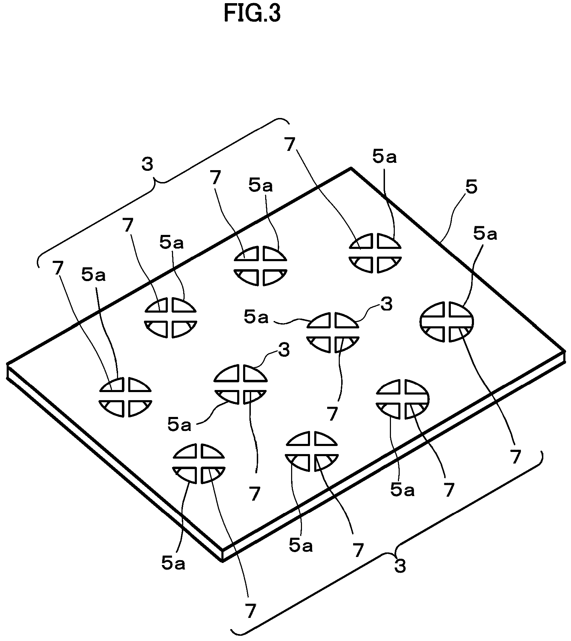

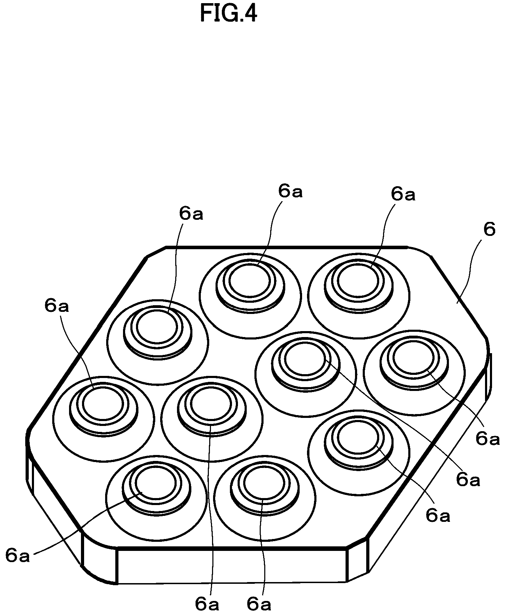

As illustrated in FIG. 3, each of the first substrates 5 has first communication holes 5a, and as illustrated in FIG. 4, each of the second substrates 6 has second communication holes 6a. In FIG. 3, a first substrate 5 is viewed from an outlet 2b side, and in FIG. 4, the second substrate 6 in the first tier is viewed from the outlet 2b side. Note that second substrates 6 in the second and subsequent tiers have different outer shapes from the second substrate 6 in the first tier. As illustrated in FIG. 2, the first substrates 5 and the second substrates 6 are stacked in such a way that the first communication holes 5a and the second communication holes 6a communicate with each other. The first communication holes 5a and the second communication holes 6a constitute chambers 2. Between a first substrate 5 and a second substrate 6, a sealing member (not illustrated) is interposed in such a way that the gas F does not leak from the chambers 2.

As illustrated in FIG. 3, each of the plurality of first substrates 5 has beams 7 each of which closes a portion of one of the first communication holes 5a. Each substance sensor 3 is disposed on a first substrate 5 and has a beam 7 as one of constituent elements of the substance sensor 3. Each substance sensor 3 detects a substance, based on a change in a vibration frequency, for example, a resonant frequency, of the beam 7 due to reaction with the substance.

As described above, the substance detecting system 1 has a configuration in which in each chamber 2, three layers of second communication holes 6a and three layers of first communication holes 5a each having a beam 7 therein are alternately stacked in the direction from the inlet 2a toward the outlet 2b (see FIG. 2). Therefore, a plurality of substance sensors 3 has a configuration in which the substance sensors 3 are arranged in three rows in the flow direction from the inlet 2a toward the outlet 2b in each chamber 2. Note that in FIG. 2, a position of the outlet 2b of each chamber 2 is a position at which a beam 7 corresponding to a substance sensor 3 in the third tier is disposed.

In each of the first substrates 5, a plurality of first communication holes 5a is disposed, and in each of the second substrates 6, a plurality of second communication holes 6a is disposed at positions in accordance with the positions at which the first communication holes 5a are disposed. Therefore, stacking the first substrates 5 and the second substrates 6 causes the first communication holes 5a and the second communication holes 6a to communicate with each other and constitute a plurality of chambers 2. In the substance detecting system 1, a plurality of chambers 2 is disposed in parallel with one another. In the present embodiment, the number of chambers 2 is ten. The ten chambers 2 are disposed in such a way that internal spaces of the chambers 2 have the same shape and size.

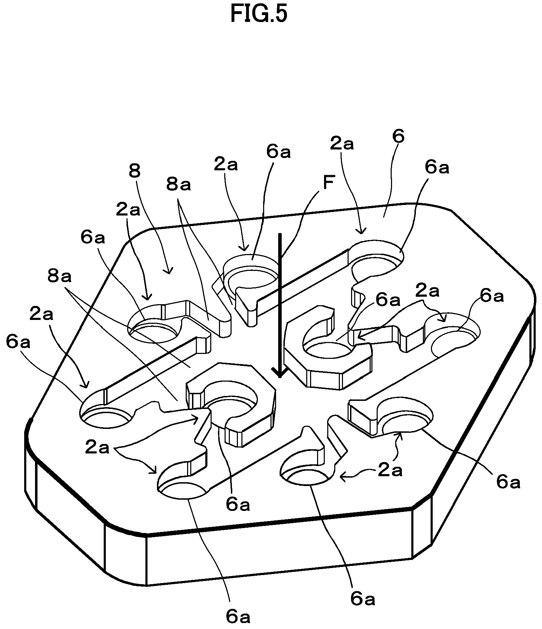

The substance detecting system 1 includes a rectifier 8 that controls flows of the gas F flowing into the plurality of chambers 2 into uniform flows and feeds the flows. As illustrated in FIG. 5, the rectifier 8 is disposed in the second substrate 6 in the first tier in which the inlets 2a of the chambers 2 are disposed. Note that FIG. 5 illustrates the second substrate 6 in the first tier viewed from the inlet 2a side, that is, the side on which the gas F flows in.

The rectifier 8 rectifies the flows of the gas F in such a way that at least one of flow rates and flow velocities of the flows of the gas F that flow into respective ones of the plurality of chambers 2 is made uniform. In the rectifier 8, a groove 8a that serves as a flow path of the gas F is disposed. By sticking a surface including the groove 8a and a substrate 10 (see FIG. 2) together, the flow path of the rectifier 8 is shaped.

As illustrated in FIG. 5, the rectifier 8 is in communication with the inlets 2a, that is, the second communication holes 6a. Into the rectifier 8, the gas F flows in at a position indicated by an arrow in FIG. 5 via an intake 10a of the substrate 10 (see FIG. 2). In the rectifier 8, the gas F having flowed in is fed to the respective second communication holes 6a via branched flow paths. Shapes of the branched flow paths of the rectifier 8 are designed in such a way that flow rates and flow velocities of the flows of the gas F that are fed to respective ones of the plurality of inlets 2a are the same.

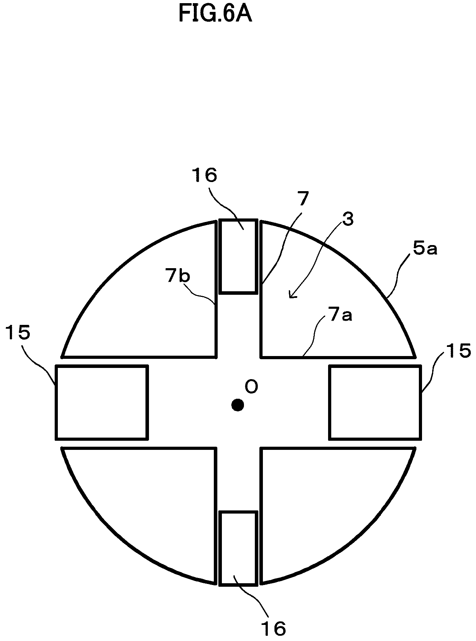

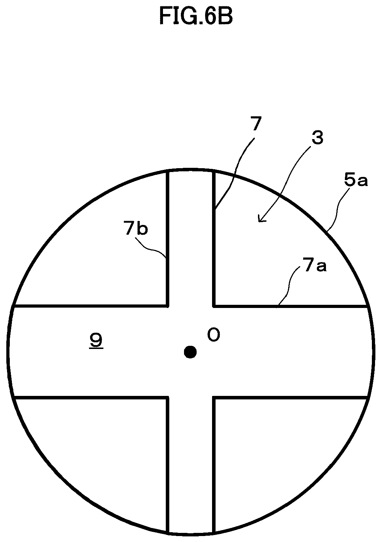

As illustrated in FIGS. 6A and 6B, in each of the first substrates 5, each of the first communication holes 5a is shaped in a cylindrical shape, and a central axis of the cylindrical first communication hole 5a is denoted by O. In addition, a beam 7 disposed in each first communication hole 5a includes a drive beam 7a and a detection beam 7b. FIG. 6A illustrates the beam 7 viewed from the outlet 2b side, that is, a downstream side of the flow of the gas F, and a surface illustrated in FIG. 6A is defined as a front surface. In addition, FIG. 6B illustrates the beam 7 viewed from the inlet 2a side, that is, an upstream side of the flow of the gas F, and a surface illustrated in FIG. 6B is defined as a back surface.

As illustrated in FIG. 6A, the drive beam 7a and the detection beam 7b are shaped in slender plate shapes having uniform widths, and the width of the drive beam 7a is larger than the width of the detection beam 7b. The drive beam 7a and the detection beam 7b are fixed to both ends of an edge of the first communication hole 5a and cross each other at right angles at the center thereof. The shape of the beam 7 when viewed from the inlet 2a side in the flow direction is rotationally symmetric, specifically two-fold rotationally symmetric, about the central axis O of the cylindrical first communication hole 5a.

A lower electrode layer and a piezoelectric element layer are layered on the entire front surface of each beam 7, and on the layers, drive electrodes 15 and detection electrodes 16 are disposed. The drive electrodes 15 are disposed at both ends of the drive beam 7a, and the detection electrodes 16 are disposed at both ends of the detection beam 7b. Although width of the drive electrodes 15 is configured to be larger than width of the detection electrodes 16 in accordance with the widths of the drive beam 7a and the detection beam 7b, the widths of the drive electrodes 15 and the detection electrodes 16 may be the same.

When a sine-wave voltage signal is applied between the drive electrodes 15 and the lower electrode layer, the piezoelectric element layer expands and contracts and the drive beam 7a vertically vibrates, and the detection beam 7b vibrates in association with the vibration of the drive beam 7a. When the detection beam 7b vibrates, voltage matching the vibration is generated in the piezoelectric element layer between the detection electrodes 16 and the lower electrode layer. A signal representing the voltage is detected as a signal indicating a vibration frequency of the beam 7.

As illustrated in FIG. 6B, a substance reaction film 9 that reacts with a substance is disposed on the entire back surface of the beam 7. When a substance adheres to the substance reaction film 9, weight of the substance reaction film 9 changes. Because of the weight change, the vibration frequency of the beam 7 changes. An example of the vibration frequency is a resonant frequency. The substance sensor 3 detects a change in the vibration frequency of the beam 7, based on the voltage signal between the detection electrodes 16 and the lower electrode layer. Note that the substance reaction film 9 does not have to be disposed (applied) on the entire back surface of the beam 7. The substance reaction film 9 is only required to be disposed on a portion of the back surface. In addition, the substance reaction film 9 may be disposed on the front surface of the beam 7. The substance reaction film 9 is only required to be disposed on either the front surface or the back surface of the beam 7.

The larger the amount of substance adhering to the substance reaction film 9 becomes, the larger the change in the vibration frequency of the beam 7 becomes. The substance sensor 3 detects the amount of substance, based on the magnitude of change in the vibration frequency of the beam 7.

As illustrated in FIG. 2, each second communication hole 6a of each second substrate 6 is shaped in a nozzle in such a way as to cause the gas F to flow out toward a substance reaction film 9. Because of this configuration, it becomes possible to increase the amount of gas F that comes into contact with the substance reaction film 9 and improve detection sensitivity to a substance.

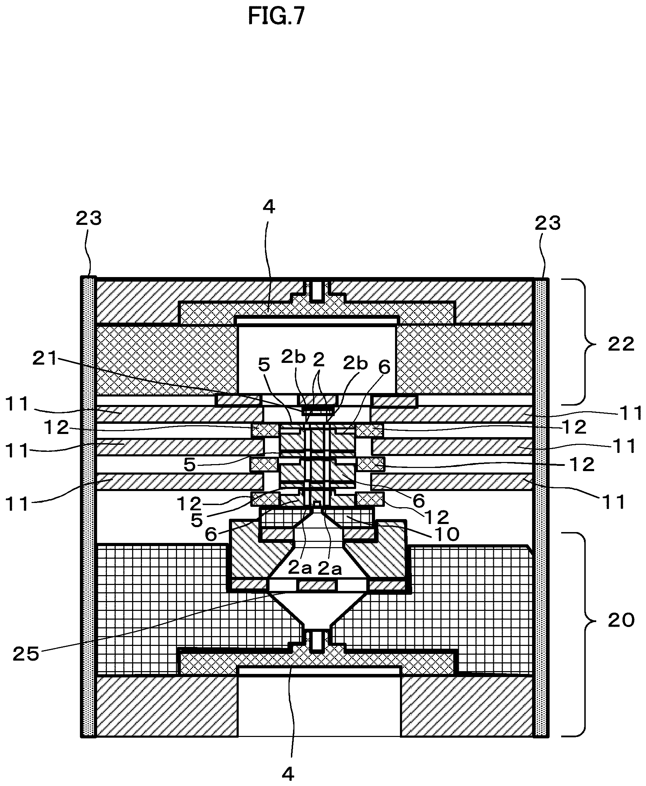

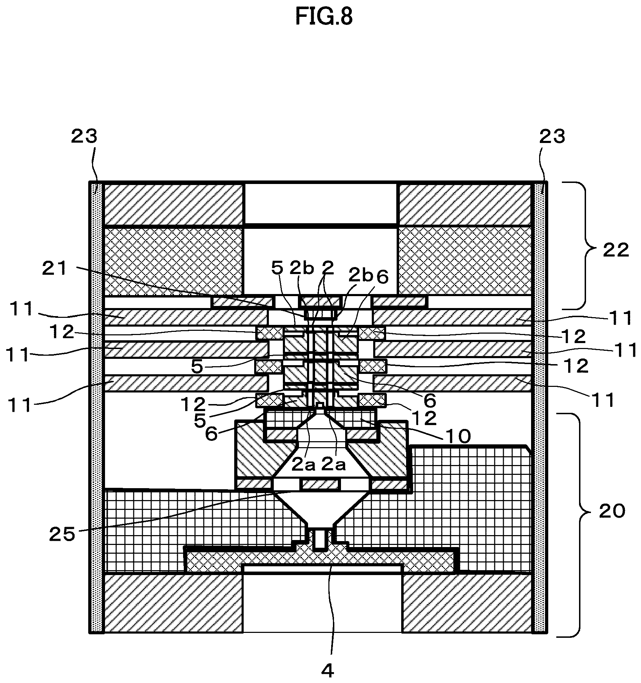

In addition, in FIG. 1, the feeder 4 is configured to be installed on the outlet 2b side. However, the present disclosure is not limited to the configuration. As illustrated in FIG. 7, the feeder 4 may be configured to be installed on both the upstream side of the inlet 2a of each chamber 2 and the downstream side of the outlet 2b of the chamber 2, and as illustrated in FIG. 8, the feeder 4 may be configured to be installed on the upstream side of the inlet 2a of each chamber 2.

Note that as illustrated in FIGS. 7 and 8, the substance detecting system 1 further includes a gas intake 20, an environment sensor 21, a gas discharger 22, and an external housing 23.

Into the gas intake 20, the feeder 4 is incorporated. The feeder 4 draws the gas F from the outside into an internal space that is in communication with the intake 10a of the substrate 10 (see FIG. 2). In the internal space, a mitigation substrate 25 is disposed. The mitigation substrate 25 mitigates the flow of the gas F drawn in from the feeder 4 and stabilizes the flow rate and the flow velocity.

The gas F fed to the internal space of the gas intake 20 is fed to the respective chamber 2 via the intake 10a of the substrate 10 and the rectifier 8 (see FIG. 5). The gas F that entered the respective chambers 2 from the inlets 2a of the chambers 2 passes through three tiers of substance sensors 3 (beams 7) and is discharged from the outlets 2b of the respective chambers 2.

The feeder 4 can also be incorporated into the gas discharger 22. The feeder 4 discharges the gas F discharged from the outlets 2b of the respective chambers 2 to the outside. Right above the outlets 2b, the environment sensor 21 is installed. Environmental information of the drawn-in gas F, such as temperature and humidity, is detected by the environment sensor 21. The environmental information detected by the environment sensor 21 is used for correction or the like of the amount of substance detected by the substance sensors 3.

The external housing 23 houses and holds the gas intake 20, the substrates 10, 11, and 12, the first substrates 5, the second substrates 6, and the gas discharger 22.

Next, operation of the substance detecting system 1 according to Embodiment 1 of the present disclosure is described.

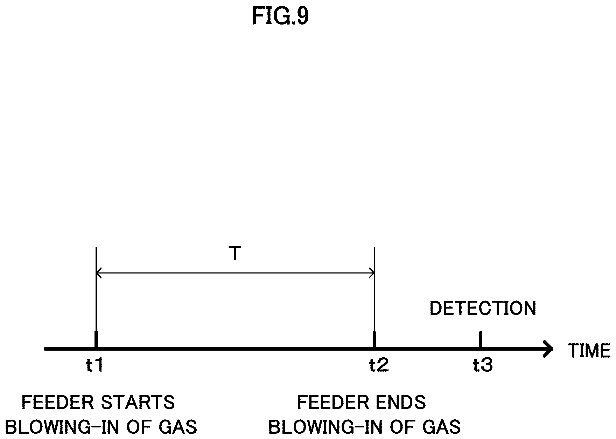

As illustrated in FIG. 9, first, at time t1, the feeder 4 starts drawing in the gas F. At and after time t1, the gas F flows into the respective chambers 2 via the rectifier 8. At time t2 after a driving time T has elapsed from time t1, the feeder 4 ends blowing-in of the gas F. At this time point, the gas F in the chambers 2 has been brought into the steady state.

After the blowing-in ended, at time t3, each of the substance sensors 3 detects a substance contained in the gas F, based on a voltage signal between the detection electrodes 16 and the lower electrode layer, that is, a signal indicating a change in the vibration frequency of the beam 7. When detection results by the plurality of substance sensors 3 are combined, a detection pattern of the amounts of substances contained in the gas F can be acquired. Since the detection pattern is equivalent to ratios of substances constituting an odor contained in the gas F, an odor including the detected substances can be accurately identified. The amounts of substances are corrected based on environmental information detected by the environment sensor 21 as needed basis.

Embodiment 2, which is according to the claimed invention.

Next, Embodiment 2 of the present disclosure is described. A configuration of a substance detecting system 1 according to the present embodiment is the same as the configuration of the substance detecting system 1 according to the above-described embodiment except a direction of a beam 7.

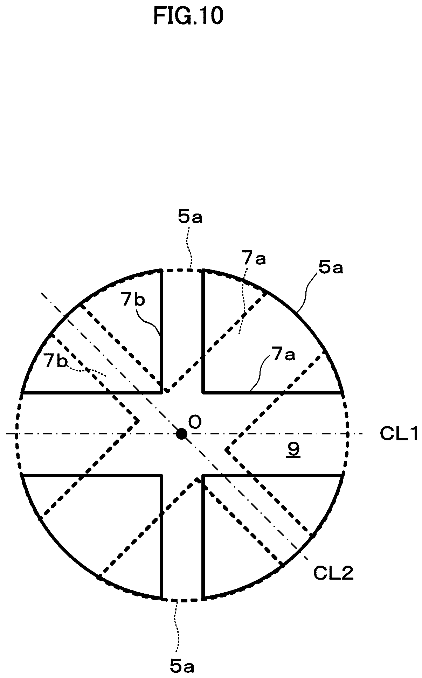

In the present embodiment, directions of beams 7 when viewed from an inlet 2a side in a flow direction are different from each other between beams 7 adjacent to each other in the flow direction. In FIG. 10, a beam 7 that can be seen from the inlet 2a side is illustrated by a solid line, and a beam 7 adjacent in the lower tier to the beam 7 illustrated by the solid line is illustrated by a dashed line. As illustrated in FIG. 10, a direction of one beam 7 when viewed from the inlet 2a side in the flow direction points in a direction rotated about a central axis O of a first communication hole 5a with respect to a direction of the other beam 7 in such a way that overlapping portions of beams 7 adjacent to each other in the flow direction from the inlet 2a toward the outlet 2b are small when viewed from the inlet 2a side.

More specifically, the beams 7 have cross shapes when viewed from the inlet 2a side in the flow direction. In addition, when viewed from the inlet 2a side in the flow direction, an angle at which, of the beams 7 adjacent to each other in the flow direction, a central line CL1 of one beam 7 and a central line CL2 of the other beam 7 intersect each other at the central axis O is 45 degrees, as illustrated in FIG. 10.

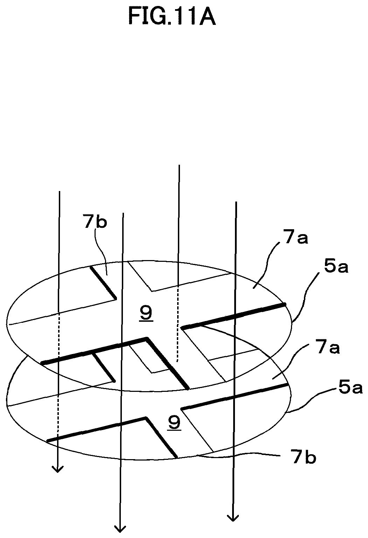

As illustrated in FIG. 11A, when the directions of the beams 7 adjacent to each other in the flow direction from the inlet 2a toward the outlet 2b are the same, a large portion of gas F that has passed through a first communication hole 5a on the upstream side passes through a first communication hole 5a on the downstream side without change. Therefore, the amount of the gas F that comes into contact with a substance reaction film 9 disposed in the first communication hole 5a on the downstream side is small.

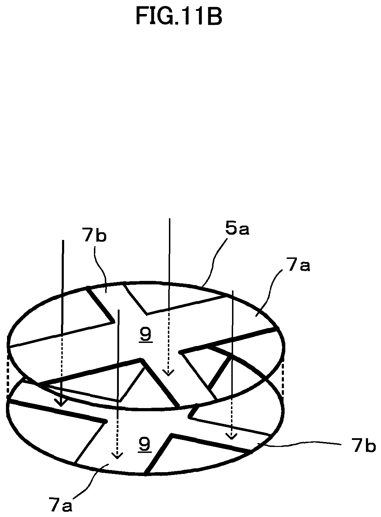

In contrast, as illustrated in FIG. 11B, a large portion of the gas F that has passed through the first communication hole 5a on the upstream side of the gas F comes into contact with the substance reaction film 9 on the beam 7 disposed in the first communication hole 5a on the downstream side. Because of this configuration, it becomes possible to increase the amount of substance that adsorbs to the substance reaction film 9 and improve detection sensitivity of the substance reaction film 9 on the beam 7 in a lower tier.

Note that even when three or more tiers of beams 7 are arranged, beams 7 adjacent to each other in the flow direction are only required to have different directions. In this case, the directions of the beams 7 may be alternately the same.

Embodiment 3, which does not belong to the claimed invention. Next, Embodiment 3 of the present disclosure is described. A configuration of a substance detecting system 1 according to the present embodiment can be configured to be the same as the configurations of the substance detecting systems 1 according to Embodiments 1 and 2 described above.

In Embodiment 1, substances detected by a plurality of substance sensors 3 that are arranged in each chamber 2 are completely different from one another. However, in the present embodiment, substances detected by a plurality of substance sensors 3 that are arranged in each chamber 2 are the same.

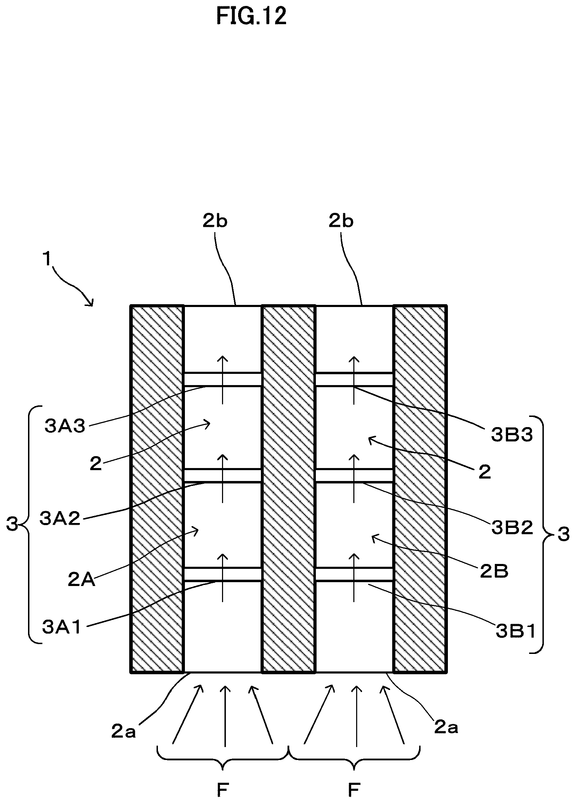

It is now assumed that as illustrated in FIG. 12, the substance detecting system 1 includes two chambers 2A and 2B as the chambers 2. Further, in the chamber 2A, three tiers of substance sensors 3A1, 3A2, and 3A3 that serve as the substance sensors 3 are arranged. In the chamber 2A, the substance sensors 3A1, 3A2, and 3A3 are arranged in this order in a flow direction from an inlet 2a toward an outlet 2b. In addition, in the chamber 2B, three tiers of substance sensors 3B1, 3B2, and 3B3 that serve as the substance sensors 3 are arranged. In the chamber 2B, the substance sensors 3B1, 3B2, and 3B3 are arranged in this order in a flow direction from an inlet 2a toward an outlet 2b.

The substance sensors 3A1, 3A2, and 3A3 detect the same substance A. The substance sensors 3B1, 3B2, and 3B3 detect the same substance B. When the substance sensors 3A1 to 3A3 and the substance sensors 3B1 to 3B3 that detect the same substances A and B in the same chambers 2A and 2B, respectively are arranged in this way, detection omission of the substances A and B can be prevented and detection precision can be improved.

When flow of the gas F is taken into consideration, a detection amount of the substance A decreases in the order of the substance sensors 3A1, 3A2, and 3A3. Thus, it may be configured such that the detection amounts of the substance A detected by the substance sensors 3A2 and 3A3 are corrected. In addition, when flow of the gas F is taken into consideration, a detection amount of the substance B decreases in the order of the substance sensors 3B1, 3B2, and 3B3. Thus, it may be configured such that the detection amounts of the substance B detected by the substance sensors 3B2 and 3B3 are corrected. As described above, in the substance detecting system 1, the substance sensors 3A1 to 3A3 can correct detected values, based on correction values that increase from the inlet 2a toward the outlet 2b, that is, in the flow direction. The same applies to the substance sensors 3B1 to 3B3.

For example, it is assumed that sensitivity of the substance sensor 3A1 in the first tier is 80%, sensitivity of the substance sensor 3A2 in the second tier is 70%, and sensitivity of the substance sensor 3A3 in the third tier is 60%. In this case, correction values of 1.14 and 1.33 can be set for the substance sensors 3A2 and 3A3, respectively. For example, a value obtained by multiplying a detected value by the substance sensor 3A2 by the correction value of 1.14 is defined as a final detected value of the substance sensor 3A2, and a value obtained by multiplying a detected value by the substance sensor 3A3 by the correction value of 1.33 is defined as a final detected value of the substance sensor 3A3.

However, among a plurality of substance sensors 3 arranged in each chamber 2, substance sensors 3 detecting the same substance and substance sensors 3 detecting different substances may coexist. That is, it may be configured such that in a plurality of substance sensors 3 arranged in a chamber 2, substance sensors 3 detecting the same substance are included, and it may also be configured such that in a plurality of substance sensors 3 arranged in a chamber 2, substance sensors 3 each detecting a different substance are included.

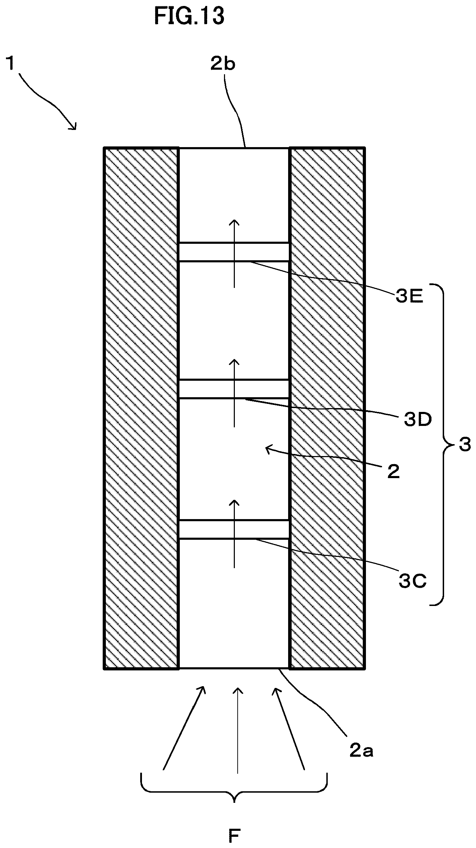

For example, as illustrated in FIG. 13, it is assumed that when a plurality of substance sensors 3 (3C, 3D, and 3E) arranged in a chamber 2 detect different substances C, D, and E, respectively, sensitivity of the substance sensor 3C in the first tier, which detects the substance C, is 80%, sensitivity of the substance sensor 3D in the second tier, which detects the substance D, is 70%, and sensitivity of the substance sensor 3E in the third tier, which detects the substance E, is 60%. In this case, correction values of 1.14 and 1.33 can be set for the substance sensors 3D in the second tier and the substance sensor 3E in the third tier, respectively. For example, a value obtained by multiplying a detected value by the substance sensor 3D in the second tier by the correction value of 1.14 is defined as a final detected value by the substance sensor 3D, and a value obtained by multiplying a detected value by the substance sensor 3E by the correction value of 1.33 is defined as a final detected value by the substance sensor 3E. For example, when a detected value by the substance sensor 3C in the first tier is 40, the detected value of 40 is directly adopted, when a detected value by the substance sensor 3D in the second tier is 50, the detected value is corrected to 57, and when a detected value by the substance sensor 3E in the third tier is 10, the detected value is corrected to 13.3. As described above, in the substance detecting system 1, the substance sensors 3 (3C, 3D, and 3E) can correct detected values, based on correction values that increase in the flow direction.

Note that substance sensors 3 detecting the same substance do not have to be arranged in the same chamber 2. When which substance each of detection results of a plurality of substance sensors 3 deals with is known, the substance sensors 3 can be freely arranged.

As described in detail in the foregoing, according to the above-described embodiments, a plurality of substance sensors 3 each of which detects a different substance is arranged in the flow direction from the inlet 2a toward the outlet 2b of each chamber 2. Thus, area of a region required for arranging the substance sensors 3 can be reduced.

Note that according to the above-described embodiments, the number of substance sensors 3 arranged along the flow direction from the inlet 2a toward the outlet 2b is set to three. However, the present disclosure is not limited to the configuration. The number of arranged substance sensors 3 may be two, or four or more.

In addition, in the above-described embodiments, each of the plurality of first substrates 5 has a beam 7 that closes a portion of each first communication hole 5a, and each substance sensor 3 is configured to detect a substance, based on a change in the vibration frequency of the beam 7 due to reaction with the substance. When configured in such a manner, a mechanism that can detect different substances contained in the gas F by feeding the gas F in the flow direction from the inlet 2a toward the outlet 2b and arranging a plurality of substance sensors 3 in the flow path of the gas F can be constructed. In addition, by employing such a configuration, the substance detecting system 1 can be miniaturized and made thinner.

In addition, in the above-described embodiments, a plurality of second substrates 6 that is the stacked substrates except the first substrates 5 is substrates having second communication holes 6a, and the second substrates 6 and the first substrates 5 are alternately stacked in this order in the flow direction when viewed from the inlet 2a side. When configured in such a manner, a manner in which the gas F comes into contact with a vicinity of a beam 7 disposed in each first communication hole 5a can be made uniform. As described above, a chamber 2 is constructed by a second substrate 6 being stacked in a preceding tier of a first substrate 5. However, when a manner in which the gas F comes into contact with the respective beams 7 can be made sufficiently uniform, the second substrates 6 do not have to be stacked.

In addition, in the above-described embodiments, a substance reaction film 9 that reacts with a substance is disposed on each beam 7, and each second communication hole 6a is shaped in a nozzle in such a way as to cause the gas F to flow out toward a substance reaction film 9. When configured in such a manner, since reaction between a substance reaction film 9 and a substance is facilitated, detection sensitivity to the substance can be improved.

In addition, in the substance detecting system 1 according to Embodiment 2 described above, directions of beams 7 when viewed from the inlet 2a side in the flow direction are different from each other between beams 7 adjacent to each other in the flow direction. When configured in such a manner, since the gas F also comes into contact with a vicinity of a substance sensor 3 on the downstream side and adherence of a substance to a substance reaction film 9 is facilitated, detection sensitivity to the substance can be improved.

In addition, according to the above-described embodiments, the shape of a beam 7 when viewed from the inlet 2a side in the flow direction is rotationally symmetric about the central axis O of a cylindrical first communication hole 5a, and regarding beams 7 adjacent to each other in the flow direction, the direction of one beam 7 when viewed from the inlet 2a side in the flow direction points in a direction rotated about the central axis O of the first communication hole 5a with respect to the direction of the other beam 7, when viewed from the inlet 2a side in the flow direction. When configured in such a manner, since the gas F is also likely to stay in a vicinity of the substance sensor 3 on the downstream side and adherence of a substance to the substance reaction film 9 is facilitated, detection sensitivity to the substance can be improved.

In addition, according to the above-described embodiments, each of the beams 7 has a cross shape when viewed from the inlet 2a side in the flow direction. In addition, when viewed from the inlet 2a side in the flow direction, an angle at which, of the beams 7, the central line CL1 of one beam 7 and the central line CL2 of the other beam 7 cross each other at the central axis is 45 degrees (see FIG. 10). When configured in such a manner, the amount of the gas F that temporarily stays in the vicinity of a substance sensor 3 can be maximized. Note that the rotation angle does not necessarily have to be 45 degrees.

In addition, in the above-described embodiments, a plurality of chambers 2 is arranged in parallel with one another. When the number of chambers 2 is configured to be increased in this way, the number of types of detectable substances can be increased. When configured in such a manner, the substance sensors 3 can be arranged in parallel and in series with one another. For example, in the above-described embodiments, the number of chambers 2 is set to 10 and the number of arranged substance sensors 3 is set to 3, and, as a result, the total number of substance sensors 3 is set to 30. In this case, the number of chambers 2 may be set to 6 and the number of arranged substance sensors 3 may be set to 5, and the number of chambers 2 may be set to 15 and the number of arranged substance sensors 3 may be set to 2. When the number of chambers 2 and the number of substance sensors 3 arranged in each chamber 2 are adjusted in this way, sizes of length, width, and height of the substance detecting system 1 can be set to appropriate values in accordance with a use.

In addition, in the above-described embodiments, the substance detecting system 1 includes a rectifier 8 that controls flows of the gas F flowing into the plurality of chambers 2 into uniform flows and feeds the flows. Since when configured in such a manner, a flow of the gas F fed to each chamber 2 can be made into a steady flow, variation in detection sensitivities of the respective substance sensors 3 can be controlled.

In addition, in the above-described embodiments, the rectifier 8 rectifies the flows of the gas F in such a way that at least one of flow rates and flow velocities of the flows of the gas F that flow into respective ones of the plurality of chambers 2 are made uniform. When configured in such a manner, a manner in which the gas F comes into contact with the respective substance sensors 3 can be made uniform.

In addition, the plurality of chambers 2 is disposed in such a way that the chambers 2 have the same shape and size. When configured in such a manner, variation in detection sensitivities of the respective substance sensors 3 can be controlled.

In addition, according to the above-described embodiments, the feeder 4 is a pump and is installed on at least one of the upstream side of the inlet 2a and the downstream side of the outlet 2b. Arrangement of the feeder 4 may be configured to be determined based on thickness that the substance detecting system 1 is required to have or a degree of variation in detected values by the plurality of substance sensors 3. In addition, the feeder 4 may be configured to be installed with respect to each chamber 2.

In addition, in the above-described embodiments, each beam 7 is configured to have a cross shape in which a drive beam 7a and a detection beam 7b cross each other at right angles. However, the present disclosure is not limited to the configuration. The drive beam 7a and the detection beam 7b are only required to cross each other. In addition, the width of the drive beam 7a and the width of the detection beam 7b may be the same, or the width of the drive beam 7a may be smaller than the width of the detection beam 7b.

In addition, it may be configured such that a drive electrode 15 is disposed at one end of the drive beam 7a and a detection electrode 16 is disposed at one end of the detection beam 7b. The widths of the drive electrodes 15 and the detection electrodes 16 may be the same.

In addition, each beam 7 does not have to have a cross shape. The beam 7 may be an elongated beam that is fixed to both ends of an edge of a first communication hole 5a. In this case, a drive electrode 15 and a detection electrode 16 are only required to be disposed at both ends or one end of the beam in parallel.

In the above-described embodiments, a shape of each first communication hole 5a is configured to be a cylindrical shape. However, the present disclosure is not limited to the configuration. A cross-sectional shape of each first communication hole 5a may be a rectangular polygonal shape or another shape.

In addition, a shape of each second communication hole 6a is configured to be a nozzle shape. However, the present disclosure is not limited to the configuration. A shape of each second communication hole 6a may be cylindrical, and a cross-sectional shape of the second communication hole 6a may be a rectangular polygonal shape or another shape.

In the above-described embodiments, the substance detecting system 1 detects a plurality of types of substances constituting an odor of the gas F. However, the present disclosure is not limited to the configuration. The present disclosure is applicable to detection of a substance that does not constitute an odor as long as the substance is a substance contained in the gas F.

As described above, the substance detecting system 1 according to the above-described embodiments can be housed in a small electronic device since a mounting area of the substance sensors 3 can be made small and the entire substance detecting system 1 can be miniaturized and made thinner. The substance detecting system 1 can be incorporated into, for example, an information device, such as a smartphone.

The foregoing describes some example embodiments for explanatory purposes. Although the foregoing discussion has presented specific embodiments, persons skilled in the art will recognize that changes may be made in form and detail without departing from the scope of the invention as defined by the appended claims. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

### Industrial Applicability

The present disclosure is applicable to detection of a substance contained in a gas.

### Reference Signs List

- 1: Substance detecting system
- 2, 2A, 2B: Chamber
- 2a: Inlet
- 2b: Outlet
- 3, 3A1, 3A2, 3A3, 3B1, 3B2, 3B3, 3C, 3D, 3E: Substance sensor
- 4: Feeder
- 5: First substrate
- 5a: First communication hole
- 6: Second substrate
- 6a: Second communication hole
- 7: Beam
- 7a: Drive beam
- 7b: Detection beam
- 8: Rectifier
- 8a: Groove
- 9: Substance reaction film
- 10: Substrate
- 10a: Intake
- 11, 12: Substrate
- 15: Drive electrode
- 16: Detection electrode
- 20: Gas intake
- 21: Environment sensor
- 22: Gas discharger
- 23: External housing
- 25: Mitigation substrate
- F: Gas

## Claims

1. A substance detecting system (1), comprising:
a chamber (2, 2A, 2B) to restrict an inflow of gas (F) to be contained inside the chamber (2, 2A, 2B) in such a way that a state of the gas (F) is brought into a steady state;
a plurality of substance sensors (3, 3A1, 3A2, 3A3, 3B1, 3B2, 3B3, 3C, 3D, 3E) to detect a substance contained in the gas (F) contained in the chamber (2, 2A, 2B); and
a feeder (4) to feed the gas (F) into the chamber (2, 2A, 2B),
wherein the plurality of the substance sensors (3, 3A1, 3A2, 3A3, 3B1, 3B2, 3B3, 3C, 3D, 3E) is arranged in a flow direction of the gas (F) from an inlet (2a) toward an outlet (2b) of the chamber (2, 2A, 2B),
the chamber (2, 2A, 2B) is constructed by substrates each having a communication hole being stacked in such a way that the communication holes communicate with each other,
a plurality of the substrates among the stacked substrates is first substrates (5) each of which has a beam (7) that closes a portion of a first communication hole (5a) serving as the communication hole,
each of the substance sensors (3, 3A1, 3A2, 3A3, 3B1, 3B2, 3B3, 3C, 3D, 3E) is configured to detect the substance, based on a change in a vibration frequency of the beam (7) due to reaction with the substance, and
**characterized in that**
directions of the beams (7) when viewed in the flow direction are different from each other between the beams (7) adjacent to each other in the flow direction.

2. The substance detecting system (1) according to claim 1, wherein
a plurality of the substrates that is the stacked substrates except the first substrates (5) is second substrates (6) each of which has a second communication hole (6a) serving as the communication hole, and
the second substrates (6) and the first substrates (5) are alternately stacked in this order in the flow direction.

3. The substance detecting system (1) according to claim 2, wherein
a substance reaction film (9) that reacts with the substance is disposed on the beam, and
each of the second communication holes (6a) is shaped in a nozzle in such a way as to cause the gas (F) to flow out toward the substance reaction film (9).

4. The substance detecting system (1) according to any one of claims 1 to 3, wherein
a shape of the beam (7) when viewed in the flow direction is rotationally symmetric about a central axis of the cylindrical first communication hole (5a), and
regarding the beams (7) adjacent to each other in the flow direction, a direction of one of the beams (7) when viewed in the flow direction points in a direction rotated about the central axis with respect to a direction of the other of the beams (7).

5. The substance detecting system (1) according to claim 4, wherein
each of the beams (7) has a cross shape when viewed in the flow direction, and
an angle at which, of the beams (7) adjacent to each other in the flow direction, a central line of one of the beams (7) and a central line of the other of the beams (7) cross each other at the central axis is 45 degrees.

6. The substance detecting system (1) according to any one of claims 1 to 5, wherein a plurality of the chambers (2, 2A, 2B) is arranged in parallel with one another.

7. The substance detecting system (1) according to claim 6, comprising a rectifier (8) to control flows of the gas (F) flowing into a plurality of the chambers (2, 2A, 2B) into uniform flows and feeds the flows.

8. The substance detecting system (1) according to claim 7, wherein the rectifier (8) rectifies a flow of the gas (F) in such a way that at least one of a flow rate and flow velocity of the gas (F) that flows into respective ones of a plurality of the chambers (2, 2A, 2B) is made uniform.

9. The substance detecting system (1) according to any one of claims 6 to 8, wherein a plurality of the chambers (2, 2A, 2B) is arranged in such a manner as to have a same shape and size.

10. The substance detecting system (1) according to any one of claims 1 to 9, wherein the feeder (4)
is a pump, and
is installed on at least one of an upstream side of the inlet (2a) and a downstream side of the outlet (2b).

11. The substance detecting system (1) according to any one of claims 1 to 9, wherein the feeder (4)
is a pump, and
is installed on both an upstream side of the inlet (2a) and a downstream side of the outlet (2b).

12. The substance detecting system (1) according to any one of claims 1 to 11, wherein in a plurality of the substance sensors (3, 3A1, 3A2, 3A3, 3B1, 3B2, 3B3, 3C, 3D, 3E) arranged in the chamber (2, 2A, 2B), substance sensors detecting a same substance are included.

13. The substance detecting system (1) according to any one of claims 1 to 11, wherein, in a plurality of the substance sensors (3, 3A1, 3A2, 3A3, 3B1, 3B2, 3B3, 3C, 3D, 3E) arranged in the chamber, substance sensors each detecting a different substance are included.

14. The substance detecting system (1) according to any one of claims 1 to 13, wherein the substance sensors (3, 3A1, 3A2, 3A3, 3B1, 3B2, 3B3, 3C, 3D, 3E) correct detected values, based on correction values that increase in the flow direction.

## Patentansprüche

1. Ein System zur Detektion von Substanzen (1), umfassend:
eine Kammer (2, 2A, 2B) zur Begrenzung eines Gaseinstroms (F), der in der Kammer (2, 2A, 2B) eingeschlossen werden soll, derart, dass der Zustand des Gases (F) in einen stationären Zustand gebracht wird;
mehrere Substanzsensoren (3, 3A1, 3A2, 3A3, 3B1, 3B2, 3B3, 3C, 3D, 3E) zum Detektieren einer im Gas (F) enthaltenen Substanz, das sich in der Kammer (2, 2A, 2B) befindet; und
eine Zuführung (4) zum Einleiten des Gases (F) in die Kammer (2, 2A, 2B),
wobei die mehrere Substanzsensoren (3, 3A1, 3A2, 3A3, 3B1, 3B2, 3B3, 3C, 3D, 3E) in einer Strömungsrichtung des Gases (F) von einem Einlass (2a) zu einem Auslass (2b) der Kammer (2, 2A, 2B) angeordnet ist,
die Kammer (2, 2A, 2B) aus Substraten aufgebaut ist, die jeweils ein Verbindungsloch aufweisen und so gestapelt sind, dass die Verbindungslöcher miteinander in Verbindung stehen,
wobei mehrere Substrate unter den gestapelten Substraten erste Substrate (5) sind, von denen jedes einen Steg (7) aufweist, der einen Abschnitt eines ersten Verbindungslochs (5a), das als Verbindungsloch dient, verschließt,
jeder der Substanzsensoren (3, 3A1, 3A2, 3A3, 3B1, 3B2, 3B3, 3C, 3D, 3E) dazu eingerichtet ist, die Substanz anhand einer Änderung der Schwingungsfrequenz des Stegs (7) infolge einer Reaktion mit der Substanz zu detektieren, und
**dadurch gekennzeichnet, dass**
sich die Richtungen der Stege (7), in Strömungsrichtung betrachtet, zwischen den in Strömungsrichtung benachbarten Stegen (7) voneinander unterscheiden.

2. Das System zur Detektion von Substanzen (1) nach Anspruch 1, wobei
mehrere der Substrate, d. h. die gestapelten Substrate mit Ausnahme der ersten Substrate (5), aus zweiten Substraten (6) bestehen, von denen jedes ein zweites Verbindungsloch (6a) aufweist, das als Verbindungsloch dient, und
die zweiten Substrate (6) und die ersten Substrate (5) in Strömungsrichtung abwechselnd in dieser Reihenfolge gestapelt sind.

3. Das System zur Detektion von Substanzen (1) nach Anspruch 2, wobei
ein Substanzreaktionsfilm (9), der mit der Substanz reagiert, auf dem Steg angeordnet ist, und
jede der zweiten Verbindungsöffnungen (6a) als Düse ausgebildet ist, sodass das Gas (F) in Richtung des Substanzreaktionsfilms (9) ausströmt.

4. Das System zur Detektion von Substanzen (1) nach einem der Ansprüche 1 bis 3, wobei
die Form des Steges (7), in Strömungsrichtung betrachtet, rotationssymmetrisch um eine Mittelachse des zylindrischen ersten Verbindungslochs (5a) ist, und
wobei bei den in Strömungsrichtung nebeneinander liegenden Stegen (7) die Richtung eines der Stege (7), in Strömungsrichtung betrachtet, um die Mittelachse gedreht ist im Vergleich zur Richtung der anderen Stege (7).

5. Das System zur Detektion von Substanzen (1) nach Anspruch 4, wobei
jeder der Stege (7) in Strömungsrichtung betrachtet eine Kreuzform aufweist und
der Winkel, unter dem sich bei den in Strömungsrichtung nebeneinander liegenden Stegen (7) die Mittellinie des einen Steges (7) und die Mittellinie des anderen Steges (7) an der Mittelachse schneiden, 45 Grad beträgt.

6. Das System zur Detektion von Substanzen (1) nach einem der Ansprüche 1 bis 5, wobei mehrere Kammern (2, 2A, 2B) parallel zueinander angeordnet sind.

7. Das System zur Detektion von Substanzen (1) nach Anspruch 6, umfassend einen Gleichrichter (8), der die in mehrere Kammern (2, 2A, 2B) strömenden Gasströme (F) in gleichmäßige Ströme umwandelt und diese zuführt.

8. Das System zur Detektion von Substanzen (1) nach Anspruch 7, wobei der Gleichrichter (8) einen Gasstrom (F) so gleichrichtet, dass zumindest entweder die Durchflussrate oder die Strömungsgeschwindigkeit des in die mehrere Kammern (2, 2A, 2B) strömenden Gases (F) vereinheitlicht wird.

9. Das System zur Detektion von Substanzen (1) nach einem der Ansprüche 6 bis 8, wobei mehrere Kammern (2, 2A, 2B) so angeordnet sind, dass sie dieselbe Form und Größe aufweisen.

10. Das System zur Detektion von Substanzen (1) nach einem der Ansprüche 1 bis 9, wobei die Zuführung (4)
eine Pumpe ist und
an mindestens einer der folgenden Stellen installiert ist: an der stromaufwärtigen Seite des Einlasses (2a) oder an der stromabwärtigen Seite des Auslasses (2b).

11. Das System zur Detektion von Substanzen (1) gemäß einem der Ansprüche 1 bis 9, wobei die Zuführung (4)
eine Pumpe ist und
sowohl auf der stromaufwärtigen Seite des Einlasses (2a) als auch auf der stromabwärtigen Seite des Auslasses (2b) installiert ist.

12. Das System zur Detektion von Substanzen (1) nach einem der Ansprüche 1 bis 11, wobei in den mehreren Substanzsensoren (3, 3A1, 3A2, 3A3, 3B1, 3B2, 3B3, 3C, 3D, 3E), die in der Kammer (2, 2A, 2B) angeordnet sind, Substanzsensoren enthalten sind, die dieselbe Substanz detektieren.

13. Das System zur Detektion von Substanzen (1) nach einem der Ansprüche 1 bis 11, wobei in den mehreren Substanzsensoren (3, 3A1, 3A2, 3A3, 3B1, 3B2, 3B3, 3C, 3D, 3E), die in der Kammer angeordnet sind, Substanzsensoren enthalten sind, die jeweils eine andere Substanz detektieren.

14. Das System zur Detektion von Substanzen (1) gemäß einem der Ansprüche 1 bis 13, wobei die Substanzsensoren (3, 3A1, 3A2, 3A3, 3B1, 3B2, 3B3, 3C, 3D, 3E) die erfassten Werte auf der Grundlage von Korrekturwerten korrigieren, die in Strömungsrichtung zunehmen.

## Revendications

1. Système de détection de substances (1), comprenant :
une chambre (2, 2A, 2B) permettant de limiter l'entrée d'un gaz (F) destiné à être contenu à l'intérieur de la chambre (2, 2A, 2B) de façon à mettre le gaz (F) dans un état stationnaire,
une pluralité de capteurs de substance (3, 3A1, 3A2, 3A3, 3B1, 3B2, 3B3, 3C, 3D, 3E) permettant de détecter une substance contenue dans le gaz (F) contenu dans la chambre (2, 2A, 2B), et
un organe d'amenée (4) permettant d'amener le gaz (F) dans la chambre (2, 2A, 2B) ;
la pluralité de capteurs de substance (3, 3A1, 3A2, 3A3, 3B1, 3B2, 3B3, 3C, 3D, 3E) étant agencés dans la direction d'écoulement du gaz (F), d'une entrée (2a) vers une sortie (2b) de la chambre (2, 2A, 2B),
la chambre (2, 2A, 2B) étant constituée de substrats, chacun présentant un trou de communication, empilés de façon que les trous de communication communiquent entre eux,
une pluralité des substrats, parmi les substrats empilés, étant des premiers substrats (5) présentant chacun une traverse (7) qui ferme une portion d'un premier trou de communication (5a) servant de trou de communication,
chacun des capteurs de substance (3, 3A1, 3A2, 3A3, 3B1, 3B2, 3B3, 3C, 3D, 3E) étant conçu pour détecter la substance en fonction d'un changement de la fréquence de vibration de la traverse (7) dû à une réaction avec la substance ;
**caractérisé en ce que**
les directions des traverses (7), vues dans la direction de l'écoulement, sont différentes concernant des traverses (7) adjacentes les unes aux autres dans la direction de l'écoulement.

2. Système de détection de substances (1) selon la revendication 1, dans lequel
une pluralité des substrats, à savoir les substrats empilés autres que les premiers substrats (5), est constituée de deuxièmes substrats (6) présentant chacun un deuxième trou de communication (6a) ayant la fonction dudit trou de communication, et
les deuxièmes substrats (6) et les premiers substrats (5) sont empilés en alternance dans cet ordre dans la direction de l'écoulement.

3. Système de détection de substances (1) selon la revendication 2, dans lequel
un film de réaction à la substance (9), qui réagit à la substance, est disposé sur la traverse, et
chacun des deuxièmes trous de communication (6a) a la forme d'une buse, permettant de faire sortir le gaz (F) en direction du film de réaction à la substance (9).

4. Système de détection de substances (1) selon l'une quelconque des revendications 1 à 3, dans lequel
la forme de la traverse (7), vue dans la direction de l'écoulement, est symétrique par rotation sur un axe central du premier trou de communication (5a) cylindrique, et
en ce qui concerne les traverses (7) adjacentes dans la direction de l'écoulement, la direction d'une des traverses (7), vue dans la direction de l'écoulement, est orientée dans une direction obtenue par rotation sur l'axe central par rapport à la direction de l'autre des traverses (7).

5. Système de détection de substances (1) selon la revendication 4, dans lequel
chacune des traverses (7) présente une forme en croix, vue dans la direction de l'écoulement, et
concernant les traverses (7) adjacentes les unes aux autres dans la direction de l'écoulement, l'angle sous lequel une ligne centrale d'une des traverses (7) et une ligne centrale de l'autre des traverses (7) se croisent au niveau de l'axe central est de 45 degrés.

6. Système de détection de substances (1) selon l'une quelconque des revendications 1 à 5, dans lequel une pluralité de chambres (2, 2A, 2B) sont agencées parallèlement les unes aux autres.

7. Système de détection de substances (1) selon la revendication 6, comprenant un organe de rectification (8) permettant de réguler les écoulements du gaz (F) entrant dans une pluralité de chambres (2, 2A, 2B) de manière à obtenir des écoulements uniformes, et d'amener lesdits flux.

8. Système de détection de substances (1) selon la revendication 7, dans lequel l'organe de rectification (8) rectifie l'écoulement du gaz (F) de façon que le débit et/ou la vitesse d'écoulement du gaz (F) entrant dans les chambres respectives d'une pluralité de chambres (2, 2A, 2B) soient uniformisés.

9. Système de détection de substances (1) selon l'une quelconque des revendications 6 à 8, dans lequel une pluralité de chambres (2, 2A, 2B) sont agencées de façon à présenter une forme et une taille identiques.

10. Système de détection de substances (1) selon l'une quelconque des revendications 1 à 9, dans lequel l'organe d'amenée (4)
est une pompe, et
est installé sur un côté amont de l'entrée (2a) et/ou sur un côté aval de la sortie (2b).

11. Système de détection de substances (1) selon l'une quelconque des revendications 1 à 9, dans lequel l'organe d'amenée (4)
est une pompe, et
est installé sur un côté amont de l'entrée (2a) et sur un côté aval de la sortie (2b).

12. Système de détection de substances (1) selon l'une quelconque des revendications 1 à 11, dans lequel une pluralité de capteurs de substance (3, 3A1, 3A2, 3A3, 3B1, 3B2, 3B3, 3C, 3D, 3E) agencés dans la chambre (2, 2A, 2B) comprend des capteurs de substance détectant une même substance.

13. Système de détection de substances (1) selon l'une quelconque des revendications 1 à 11, dans lequel une pluralité de capteurs de substance (3, 3A1, 3A2, 3A3, 3B1, 3B2, 3B3, 3C, 3D, 3E) agencés dans la chambre comprend des capteurs de substance détectant chacun une substance différente.

14. Système de détection de substances (1) selon l'une quelconque des revendications 1 à 13, dans lequel les capteurs de substances (3, 3A1, 3A2, 3A3, 3B1, 3B2, 3B3, 3C, 3D, 3E) corrigent les valeurs détectées en fonction de valeurs de correction qui augmentent dans la direction de l'écoulement.
